# EUROPEAN PATENT APPLICATION

(11) **EP 2 662 067 A1**
(43) Date of publication of application: **13.11.2013**
(21) Application number: 12003764.3
(22) Date of filing: 11.05.2012
(51) Int. Cl.: A61K 6/083, A61K 6/087

(54) **Dental composition**

(71) Applicant: Dentsply DeTrey GmbH, 78467 Konstanz (DE)
(72) Inventor: Klee, Prof. Dr. Joachim, 78315 Radolfzell (DE); Maier, Maximilian, 40213 Düsseldorf (DE); Fik, Christoph P., 78256 Steisslingen (DE); Ritter, Prof. Dr. Helmut, 42111 Wuppertal (DE)
(74) Representative: Hartz, Nikolai

(57) **Abstract**

Dental composition comprising
(i) a polymerizable poly(ethylenimine) derivative, which contains repeating units of the following formula (I) 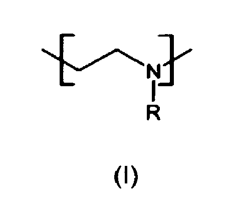 wherein the R, which are independent from each other, are selected from one or more groups of the following formula (II): 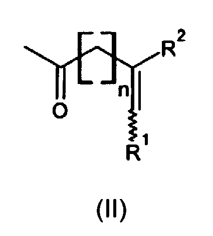 wherein
R¹
is a hydrogen atom, an alkyl group, a group -(CH₂)ₒCOOM', wherein M' is a hydrogen atom or a monovalent cation, and o is an integer of from 0 to 6;
R²
is a hydrogen atom, an alkyl group, or a group -(CH₂)ₚCOOM", wherein M" is a hydrogen atom or a monovalent cation, and p is an integer of from 0 to 6;
n
is 0 or 1;

and optionally a hydrogen atom, a further repeating unit of formula (I), and one or more groups of the following formula (III): 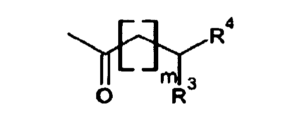 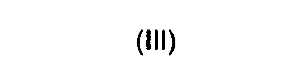 wherein
R³
is a group -(CH₂)_{q}COOM''', wherein Mʺʹ is a hydrogen atom or a monovalent cation, and q is an integer of from 0 to 6;
R⁴
is a hydrogen atom, an alkyl group, or a group -(CH₂)ᵣCOOM'''', wherein Mʺʺ is a hydrogen atom or a monovalent cation, and r is an integer of from 0 to 6;
m
is an integer of 0 to 4;
provided that when none of R in any of the repeating groups of formula (I) of the polymerizable poly(ethylenimine) derivative is a group of formula (II) wherein R¹ is a group -(CH₂)ₒCOOM' or R² is a group -(CH₂)ₚCOOM'', then at least one R must be a group of formula (III);
(ii) an initiator system;
(iii) water; and
(iv) optionally a particulate filler.

## Description

### Field of the Invention

The present invention relates to a dental composition comprising a polymerizable poly(ethylenimine) derivative. Moreover, the present invention relates to process for preparing a dental composition which comprises the polymerizable poly(ethylenimine) derivative of the present invention.

According to the present invention, it is possible to conveniently and efficiently obtain a water miscible, hydrolysis-stable and polymerizable polymer for a dental composition, notably a glass ionomer cement, at a high molecular weight, which is resistant to acidic media and capable of further crosslinking providing improved storage stability and long-term mechanical resistance of a dental ionomer cement.

### Background of the Invention

Dental compositions are used for restoring the function, morphology and integrity of dental structures damaged by physical damage or caries-related decay of enamel and/or dentin. Common to all dental compositions is that they require high biocompatibility, resistance to the severe conditions present in the oral cavity, particularly over a longer period of time.

Glass ionomer cements (GIC), which are cured by an acid-base reaction between silicate glass powder and a polyalkenoic acid, provide high biocompatibility, good direct adhesion to the dental hard tissues and cariostatic properties through the release of fluoride ions and are widely used as direct dental restorative materials. However, conventional glass ionomer cements are relatively brittle due to low flexural strength properties. Therefore, conventional glass ionomer cements cannot be used successfully for permanent dental restorations.

The resistance of glass ionomer cements to mechanical stress may be improved by the choice of the polymer for a glass ionomer cement. For example, a polymer for a glass ionomer cement, which has polymerizable moieties as pendant groups can be crosslinked to increase the mechanical resistance of the resulting glass ionomer cement.

Moreover, for the purpose of the cement reaction as well as for providing adhesive properties of the dental composition to hard dental tissue, acidic groups in the polymer are required. However, in the presence of water, acidic groups accelerate hydrolysis of pendant functional groups linked to the polymer backbone by hydrolysable groups such as ester groups. Thus, a polymer to be used in a dental composition desirably has a high stability with regard to hydrolysis in order to avoid degradation of the composition during storage or when applied to hard dental tissue.

Japanese Patent Publication No. 2005-65902A discloses a dental adhesive composition comprising, as a polymerizable monomer containing a particular carboxylic acid, a carboxylic acid compound having a (meth)acryloyl group and a carboxyl group which are bound to an aromatic group. However, such a polymerizable monomer having an ester group quickly degrades in an aqueous acidic medium.

Chen et al. and Nesterova et al. (Chen et al., J. Appl. Polym. Sci., 109 (2008) 2802-2807; Nesterova et al., Russian Journal of Applied Chemistry, 82 (2009) 618-621) disclose copolymers of N-vinylformamide with acrylic acid and/or methacrylic acid, respectively. However, none of these documents mentions the introduction of a further polymerizable moiety into the copolymer.

WO2003/011232 discloses water-based medical and dental cements that can be post-polymerized after the cement reaction. The dental cements consist of two separate polymers, wherein one of the polymers has a pendant post-polymerizable moiety linked to the polymer through an ester bond. However, this ester bond between the polymer and the polymerizable moieties is again prone to hydrolytic cleavage in acidic media. Moreover, crosslinking of the glass ionomer may lead to the shrinkage of the dental composition in particular when the molecular weight of the crosslinking polymer is low.

### Summary of the Invention

The present invention provides a dental composition comprising
(i) a polymerizable poly(ethylenimine) derivative, which contains repeating units of the following formula (I) 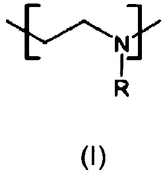 wherein the R, which are independent from each other, are selected from one or more groups of the following formula (II): 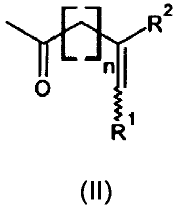 wherein
   - R¹: is a hydrogen atom, an alkyl group, a group -(CH₂)ₒCOOM', wherein M' is a hydrogen atom or a monovalent cation, and o is an integer of from 0 to 6;
   - R²: is a hydrogen atom, an alkyl group, or a group -(CH₂)ₚCOOM", wherein M" is a hydrogen atom or a monovalent cation, and p is an integer of from 0 to 6;
   - n: is 0 or 1;
   and optionally a hydrogen atom, a further repeating unit of formula (I), and one or more groups of the following formula (III): 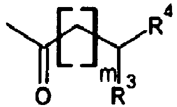 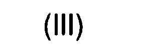
   wherein
   - R³: is a group -(CH₂)_{q}COOM"', wherein M'" is a hydrogen atom or a monovalent cation, and q is an integer of from 0 to 6;
   - ⁴: R is a hydrogen atom, an alkyl group, or a group -(CH₂)ᵣCOOM"", wherein M"" is a hydrogen atom or a monovalent cation, and r is an integer of from 0 to 6;
   - m: is an integer of 0 to 4;
   provided that when none of R in any of the repeating groups of formula (I) of the polymerizable poly(ethylenimine) derivative is a group of formula (II) wherein R¹ is a group -(CH₂)ₒCOOM' or R² is a group -(CH₂)ₚCOOM", then at least one R must be a group of formula (III);
(ii) an initiator system;
(iii) water; and
(iv) optionally a particulate filler.

The present inventors have recognized that resin reinforced dental glass ionomer cements are subject to deterioration during storage or after curing in the mouth of the patient. The present inventors have further recognized that the deterioration is due to hydrolytic degradation of the resin component conventionally containing hydrolyzable moieties. The present inventors have then recognized that by using a specific process for the preparation of a polymer, an improved water-soluble, hydrolysis-stable, polymerizable polymer may be prepared at a high molecular weight which overcomes the drawbacks of conventional resin reinforced glass ionomer cements known from the prior art. In particular, the present invention is based on the recognition that the introduction of amino group containing repeating units into the backbone of the polymer opens up the possibility to provide high molecular weight copolymers which may be easily and efficiently functionalized by the introduction of polymerizable pendant groups linked to the backbone by hydrolysis stable linking groups so that the disadvantages of conventional polymerizable resin components may be avoided.

Moreover, the present invention provides a process for preparing a dental composition which comprises reacting a poly(ethylenimine) derivative, which contains repeating units of the following formula (I) 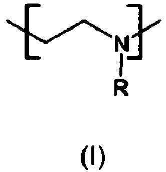 wherein R is a hydrogen atom with a compound of the following formula (IV) 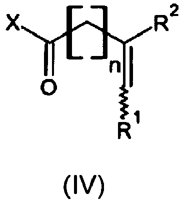 wherein R¹, R² and n are as defined in claim 1 and X is a leaving group or forms together with R¹ or R² a cyclic carboxylic anhydride, and optionally with a compound of the following formula (V) 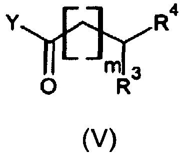 wherein R³, R⁴ and m are as defined in claim 1 and Y is a leaving group or forms together with R³ or R⁴ a cyclic carboxylic anhydride.

The process of the present invention provides a polymer useful in a dental composition, notably a glass ionomer cement, which is hydrolysis-stable and can be polymerized to yield a dental glass ionomer cement of improved mechanical resistance. The polymer may be provided with acidic groups resulting in an excellent adhesion to dental hard tissue. Moreover, since the process of the present invention provides a polymer having a high molecular weight, any polymer shrinkage during the curing reaction may be easily controlled

### Detailed Description of Preferred Embodiments

The preset invention provides a dental composition. The dental composition may be a dental material to be used in the oral cavity. Dental compositions according to the present invention are useful as restorative and filling materials, luting cements, adhesive cements, base or orthodontic cements, cavity liners and bases, and pit and fissure sealants. Certain embodiments of the polymerizable poly(ethylenimine) derivative of the present invention may also be used in other types of dental compositions such as dental composites of dental bonding agents.

The dental composition comprises a polymerizable poly(ethylenimine) derivative having at least one polymerizable double bond. The polymerizable poly(ethylenimine) derivative may be a linear or branched poly(ethylenimine) derivative.

A linear poly(ethylenimine) derivative may be obtained by acid catalyzed polymerization of a suitable oxazoline derivative such as 2-ethyl-2-oxazoline followed by hydrolytic cleavage of the acyl groups formed during the polymerization reaction. Alternatively, the oxazoline derivative may be an oxazoline derivative having a substituent which forms a (meth)acrylamide group during polymerization.

A branched poly(ethylenimine) derivative may be obtained by cationic polymerization of aziridine.

A polymerizable poly(ethylenimine) derivative of the present invention contains repeating units of the following formula (I):

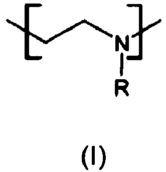

Preferably, the poly(ethylenimine) derivative is an oligomer or polymer which contains at least 80 percent by weight based on the entire polymer of repeating units of the formula (I). More preferably, the poly(ethylenimine) derivative is an oligomer or polymer which contains essentially 100 percent by weight based on the entire polymer of repeating units of the formula (I). An oligomer or polymer according to the present invention preferably has an average molecular weight Mw in the range of from 300, 500 or in particular 10,000 to 1,000,000 Da. More preferably, the average molecular weight Mw of a polymer is in the range of from 100,000 to 700,000 Da.

According to a specific embodiment, the poly(ethylenimine) derivative of the present invention is an oligomer preferably having an average molecular weight Mw in the range of from 300 to 3000 Da, more preferably in the range of from 500 to 2500.

In the formula (I), the R, which are independent from each other, are selected from one or more groups of formula (II) and optionally a hydrogen atom, a further repeating unit of formula (I), and one or more groups of formula (III).

When the poly(ethylenimine) derivative is a linear poly(ethylenimine) oligomer or polymer, R cannot be a further repeating unit of formula (I) when the repeating unit of formula (I) is located between non-hydrogen atoms.

When the poly(ethylenimine) derivative is a branched poly(ethylenimine) polymer, at least one R is a further repeating unit of formula (I) for any repeating unit of formula (I) which is not located between non-hydrogen atoms. At least one R in a poly(ethylenimine) derivative is a group of the following formula (II).

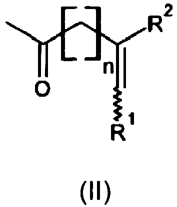

In formula (II), R¹ is a hydrogen atom, an alkyl group, a group -(CH₂)ₒCOOM', wherein M is a hydrogen atom or a monovalent cation, and o is an integer of from 0 to 6, or a further group of formula (II). According to a preferred embodiment, R¹ is a hydrogen atom.

In formula (II), R² is a hydrogen atom, an alkyl group, a group -(CH₂)ₚCOOM", wherein M" is a hydrogen atom or a monovalent cation, and p is an integer of from 0 to 6, or a further group of formula (II). According to a preferred embodiment, R² is an alkyl group. According to a further preferred embodiment, R² is a group -(CH₂)ₚCOOM", wherein M" is a hydrogen atom or a monovalent cation, and p is an integer of from 0 to 6.

In formula (II), n is 0 or 1. According to a generic embodiment, n is 0. Accordingly, a group of formula (II) may be a (meth)acryl group. According to a further generic embodiment, n is 1. Accordingly, a group of formula (II) may be an itaconic acid derivative.

Optionally further R are selected from a hydrogen atom, a further repeating unit of formula (I), and one or more groups of the following formula (III): 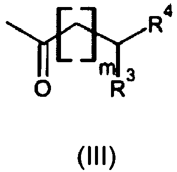 wherein
- R³: is a group -(CH₂)_{q}COOM"', wherein M"' is a hydrogen atom or a monovalent cation, and q is an integer of from 0 to 6;
- R⁴: is a hydrogen atom, an alkyl group, or a group -(CH₂)ᵣCOOM"", wherein M"" is a hydrogen atom or a monovalent cation, and r is an integer of from 0 to 6;
- m: is an integer of 0 to 4.

When none of R in any of the repeating groups of formula (I) of the polymerizable poly(ethylenimine) derivative is a group of formula (II) wherein R¹ is a group - (CH₂)ₒCOOM' or R² is a group -(CH₂)ₚCOOM", then at least one R must be a group of formula (III).

The monovalent cation in M', M", M"', and M"" may be selected from an alkali metal cation such as lithium, sodium or potassium, or an ammonium ion which may have one to four organic substituents such as optionally substituted alkyl, aryl or aralkyl substituents.

In a preferred embodiment, o, p, q, and r, which are independent from each other, is 0 to 3.

Since the polymerizable poly(ethylenimine) derivative contains an acidic carboxylic acid group, an additional curing mechanism may be employed which is based on a reaction between the polymerizable poly(ethylenimine) derivative and a reactive particulate filler, such as a powdered metal oxide or hydroxide, mineral silicate, or ion leachable glass or ceramic. Preferably, such a glass-ionomer cement is formed by reacting a polymer according to the present invention with a fluoroaluminosilicate glass (FAS glass), and optionally a further ionic polymer, e.g. a polyalkenoic acid.

In case the poly(ethylenimine) derivative is a polymer which contains at least 80 percent by weight based on the entire polymer of repeating units of the formula (I), the other repeating units may be repeating units of formula (I) wherein R is an organic residue which is different from a further repeating unit of formula (I) or a group of the following formula (II). As examples of such other repeating units may be mentioned repeating units of formula (I) wherein R is an alkyl group or an acyl group.

A polymerizable poly(ethylenimine) derivative having at least one polymerizable double bond may be prepared by a process for preparing a dental composition which comprises reacting a poly(ethylenimine) derivative, which contains repeating units of the following formula (I) 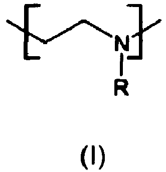 wherein R is a hydrogen atom with a compound of the following formula (III) 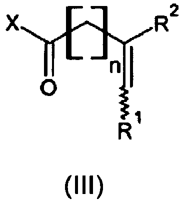 wherein R¹, R² and n are as defined above and X is a leaving group or forms together with R¹ or R² a cyclic carboxylic anhydride. Preferably, the compound of formula (III) is selected from succinic anhydride and methacrylic anhydride.

The poly(ethylenimine) derivative, which contains repeating units of the formula (I) wherein R is a hydrogen atom, may be a linear poly(ethylenimine) obtainable by hydrolyzing poly(2-ethyl-2-oxazolin). Alternatively, the branched poly(ethylenimine) derivative, which contains repeating units of the formula (I) wherein R is a further group of the formula (I)hydrogen atom, may be obtainable by polymerizing aziridine.

The reaction may be carried out in the presence of a suitable solvent such as an alcohol selected from chloroform or methylene chloride.

The reaction temperature may be selected between -10°C to the boiling point of the solvent and is typically adjusted in a range of from 0°C to 30 °C.

Alternatively, a poly(ethylenimine) derivative having a alkylacryl amide group may be prepared by cationic polymerization of an alkenyl substituted oxazoline derivative according to the following scheme: 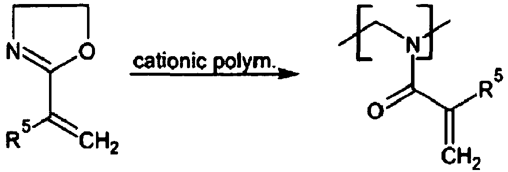 wherein R⁵ is a C₁₋₆ alkyl group, preferably a C₁₋₄ alkyl group. In case R⁵ is a methyl group, a methacryl group may be introduced according to the following scheme:

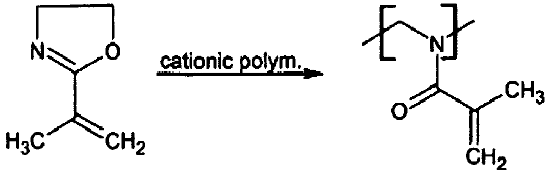

Moreover, a poly(ethylenimine) derivative having a carboxylic acid group bonded to the backbone via an amide bond may also be prepared by cationic polymerization of a substituted oxazoline derivative followed by hydrolysis according to the following scheme: 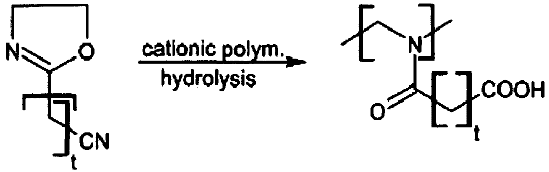 wherein t is an integer of from 1 to 6. In case t is 1, an acidic side chain may be introduced according to the following scheme:

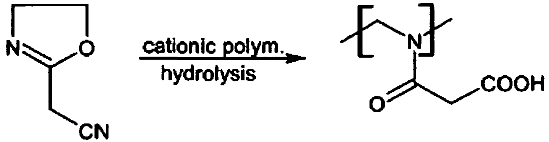

The dental composition of the present invention further comprises an initiator system. An initiator system is capable of initiating a copolymerization reaction. The initiator system may be based on a redox initiator or on a photoinitiator.

In case the dental composition contains a redox initiator, the amount of reducing agent and oxidizing agent should be sufficient to provide the desired degree of polymerization. Preferably, the dental composition of the invention contains a combined weight of about 0.01 to about 10%, more preferably about 0.2 to about 5%, and most preferably about 0.3 to about 3% of the reducing agent and oxidizing agent, based on the total weight (including any water) of the mixed but unset components. The reducing agent or the oxidizing agent can be microencapsulated as described in U.S. Pat. No. 5,154,762. This will generally enhance shelf stability of the cement parts and if necessary permit packaging both the reducing agent and oxidizing agent together. Water-soluble and water-insoluble encapsulants can be employed. Suitable encapsulating materials include cellulosic materials as cellulose acetate, cellulose acetate butyrate, ethyl cellulose, hydroxymethyl cellulose and hydroxyethyl cellulose being preferred. Other encapsulants include polystyrene, copolymers of polystyrene with other vinylic monomers and polymethylmethacrylate, copolymers of methyl methacrylate with other ethylenically-unsaturated monomers. Preferred encapsulants are ethylcellulose and cellulose acetate butyrate. By varying the choice of encapsulant and the encapsulation conditions, the onset of curing can be tailored to start at times ranging from seconds to minutes. The ratio of amount of encapsulant to activator generally ranges from 0.5 to about 10 and preferably from about 2 to about 6.

Suitable oxidizing agents (initiators) include peroxides such as cumene hydroperoxide, benzoyl peroxide and tert-butyl hydroperoxide, ferric chloride, hydroxylamine (depending upon the choice of reducing agent), perboric acid and its salts, and salts of a permanganate or persulfate anion. Preferred oxidizing agents are peroxides, potassium persulfate, ammonium persulfate and hydrogen peroxide.

Suitable reducing agents (activators) include ascorbic acid, ferrous chloride, ferrous sulfate, hydrazine, hydroxylamine (depending upon the choice of oxidizing agent) oxalic acid, thiourea, benzyl thiourea, and salts of a dithionite or sulfite anion. Preferred reducing agents include ascorbic acid and ferrous sulfate.

A photoinitiator should be capable of promoting polymerization of the polymerizable groups on exposure to light of a suitable wavelength and intensity. The photoinitiator preferably is sufficiently shelf-stable and free of undesirable coloration to permit its storage and use under typical dental conditions. Visible light photoinitiators are preferred. The photoinitiator preferably is water-soluble or water-miscible. Suitable visible light-induced and ultraviolet light-induced initiators include an alpha-diketone (e.g., camphorquinone) with or without additional hydrogen donors (such as sodium benzene sulfinate, amines and amine alcohols). The photoinitiator may be present in an amount sufficient to provide the desired rate of photopolymerization. This amount will be dependent in part on the light source, the thickness of the layer to be exposed to radiant energy and the extinction coefficient of the photoinitiator. Preferably, the dental composition of the invention will contain about 0.01 to about 5%, more preferably from about 0.1 to about 2% photoinitiator, based on the total weight (including any water) of the mixed but unset components.

The dental composition of the present invention may further comprise water or a water soluble solvent. Water serves as a medium facilitating the transport of ions, thereby allowing the acid-base chemical cure setting reaction between an ionomer and a filler to occur.

The dental composition of the present invention may further comprise a particulate filler. The particulate filler may be a reactive particulate filler or a non-reactive particulate filler. Examples of reactive particulate filler materials include materials commonly known in the art of dental compositions such as calcium or strontium-containing and aluminum-containing materials. Preferably, particulate reactive fillers contain leachable fluoride ions. Specific examples of particulate reactive fillers are selected from calcium alumino silicate glass, calcium alumino fluorosilicate glass, calcium aluminumfluoroborosilicate glass, strontium aluminosilicate glass, strontium aluminofluorosilicate glass, strontium aluminofluoroborosilicate glass. Suitable particulate reactive fillers further include metal oxides such as zinc oxide and magnesium oxide, and ion-leachable glasses, e.g., as described in US-A 3,655,605, US-A 3,814,717, US-A 4,143,018, US-A 4,209,434, US-A 4,360,605 and US-A 4,376,835.

Suitable non-reactive fillers may be selected from fillers currently used in dental restorative compositions. The filler may have a unimodal or polymodal (e.g., bimodal) particle size distribution. The filler can be an inorganic material. It can also be a crosslinked organic material that is insoluble in the polymerizable resin, and is optionally filled with inorganic filler. The filler can be radiopaque, radiolucent or non-radiopaque. Examples of suitable non-reactive inorganic fillers are naturally-occurring or synthetic materials such as quartz, nitrides such as silicon nitride, glasses derived from, for example Ce, Sb, Sn, Zr, Sr, Ba and Al, colloidal silica, feldspar, borosilicate glass, kaolin, talc, titania, and zinc glass, and submicron silica particles such as pyrogenic silicas. Examples of suitable non-reactive organic filler particles include filled or unfilled pulverized polycarbonates or polyepoxides. Preferably the surface of the filler particles is treated with a coupling agent in order to enhance the bond between the filler and the matrix. The use of suitable coupling agents includes gamma-methacryloxypropyltrimethoxysilane, gamma-mercaptopropyltriethoxysilane, gamma-aminopropyltrimethoxysilane, and the like.

The particulate filler usually has an average particle size of from 0.005 to 100 µm, preferably of from 0.01 to 40 µm as measured, for example, by electron microscopy or by using a conventional laser diffraction particle sizing method as embodied by a MALVERN Mastersizer S or MALVERN Mastersizer 2000 apparatus. The particulate filler may be a multimodal particulate reactive filler representing a mixture of two or more particulate fractions having different average particle sizes. The particulate reactive filler may also be a mixture of particles of different chemical composition. In particular, it is possible to use a mixture of a particulate reactive material and a particulate non-reactive material. The particulate reactive filler may be surface modified by a surface modifying agent.

To effect cross-linking or additional cross-linking of the cement, one or more comonomers may be included in the dental composition. Suitable comonomers contain at least one polymerizable functional group. Suitable polymerizable functional groups are ethylenically unsaturated groups (e. g. alkenyl groups and preferably vinyl groups). Ethylenically unsaturated groups are polymerizable by a free radical mechanism. Preferred examples are alpha,beta-unsaturated monomers selected from acrylates and methacrylates such as methyl acrylate, methyl methacrylate, ethyl acrylate, ethyl methacrylate, propyl acrylate, propyl methacrylate, isopropyl acrylate, isopropyl methacrylate, 2-hydroxyethyl acrylate, 2-hydroxyethyl methacrylate (HEMA), hydroxypropyl acrylate, hydroxypropyl methacrylate, tetrahydrofurfuryl acrylate, tetrahydrofurfuryl methacrylate, glycidyl acrylate, glycidyl methacrylate, the diglycidyl methacrylate of bis-phenol A ("bis-GMA"), glycerol mono- and di- acrylate, glycerol mono- and di- methacrylate, ethyleneglycol diacrylate, ethyleneglycol dimethacrylate, polyethyleneglycol diacrylate (where the number of repeating ethylene oxide units vary from 2 to 30), polyethyleneglycol dimethacrylate (where the number of repeating ethylene oxide units vary from 2 to 30 especially triethylene glycol dimethacrylate ("TEGDMA"), neopentyl glycol diacrylate, neopentylglycol dimethacrylate, trimethylolpropane triacrylate, trimethylol propane trimethacrylate, mono-, di-, tri-, and tetra- acrylates and methacrylates of pentaerythritol and dipentaerythritol, 1,3-butanediol diacrylate, 1,3-butanediol dimethacrylate, 1,4-butanedioldiacrylate, 1,4-butanediol dimethacrylate, 1,6-hexane diol diacrylate, 1,6-hexanediol dimethacrylate, di-2-methacryloyloxethyl hexamethylene dicarbamate, di-2-methacryloyloxyethyl trimethylhexanethylene dicarbamate, di-2-methacryloyl oxyethyl dimethylbenzene dicarbamate, methylene-bis-2-methacryloxyethyl-4-cyclohexyl carbamate, di-2-methacryloxyethyl-dimethylcyclohexane dicarbamate, methylene-bis-2-methacryloxyethyl-4-cyclohexyl carbamate, di-1-methyl-2-methacryloxyethyl-trimethyl-hexamethylene dicarbamate, di-1-methyl-2-methacryloxyethyl-dimethylbenzene dicarbamate, di-1-methyl-2-methacryloxyethyl-dimethylcyclohexane dicarbamate, methylene-bis-1-methyl-2-methacryloxyethyl-4-cyclohexyl carbamate, di-1-chloromethyl-2-methacryloxyethyl-hexamethylene dicarbamate, di-1-chloromethyl-2-methacryloxyethyl-trimethylhexamethylene dicarbamate, di-1-chloromethyl-2-methacryloxyethyl-dimethylbenzene dicarbamate, di-1-chloromethyl-2-methacryloxyethyl-dimethylcyclohexane dicarbamate, methylene-bis-2-methacryloxyethyl-4-cyclohexyl carbamate, di-1-methyl-2-methacryloxyethyl-hexamethylene dicarbamate, di-1-methyl-2-methacryloxyethyl-trimethylhexamethylene dicarbamate, di-1-methyl-2-methacryloxyethyl-dimethylbenzene dicarbamate, di-1-methyl-2-metha-cryloxyethyl-dimethylcyclohexane dicarbamate, methylene-bis-1-methyl-2-methacryloxyethyl-4-cyclohexyl carbamate, di-1-chloromethyl-2-methacryloxyethyl-hexamethylene dicarbamate, di-1-chloromethyl-2-methacryloxyethyl-trimethylhexamethylene dicarbamate, di-1-chloromethyl-2-methacryloxyethyl-dimethylbenzene dicarbamate, di-1-chloromethyl-2-methacryloxyethyl-dimethylcyclohexane dicarbamate, methylene-bis-1-chloromethyl-2-methacryloxyethyl4-cyclohexyl carbamate, 2,2'-bis(4-methacryloxyphenyl)propane, 2,2'bis(4-acryloxyphenyl)propane, 2,2'-bis[4(2-hydroxy-3-methacryloxy-phenyl)]propane, 2,2'-bis[4(2-hydroxy-3-acryloxy-phenyl)propane, 2,2'-bis(4-methacryloxyethoxyphenyl)propane, 2,2'-bis(4-acryloxyethoxyphenyl)propane, 2,2'-bis(4-methacryloxypropoxyphenyl)propane, 2,2'-bis(4-acryloxypropoxyphenyl)propane, 2,2'-bis(4-methacryloxydiethoxyphenyl)propane, 2,2'-bis(4-acryloxydiethoxyphenyl)propane, 2,2'-bis[3(4-phenoxy)-2-hydroxypropane-1-methacrylate]propane, and 2,2'-bis[3(4-phenoxy)-2-hydroxypropane-1-acryalte]propane, may be mentioned. Other suitable examples of polymerizable components are isopropenyl oxazoline, vinyl azalactone, vinyl pyrrolidone, styrene, divinylbenzene, urethane acrylates or methacrylates, epoxy acrylates or methacrylates and polyol acrylates or methacrylates. Mixtures of alpha,beta-unsaturated monomers can be added if desired.

The dental compositions of the present invention may further contain solvents, pigments, free radical scavengers, polymerization inhibitors, reactive and nonreactive diluents e.g., 2-hydroxyethyl methacrylate (HEMA), hydroxypropyl methacrylate, bisacrylamides such as N,N'-diethyl-1,3-bisacrylamido-propan (BADEP), 1,3-bisacrylamido-propan (BAP), and 1,3-bisacrylamido-2-ethyl-propan (BAPEN), surfactants (such as to enhance solubility of an inhibitor e. g., polyoxyethylene), coupling agents to enhance reactivity of fillers e.g.,3-(trimethoxysilyl) propyl methacrylate, and rheology modifiers.

An example of a suitable free radical scavenger is 4-methoxyphenol. An example of a suitable inhibitor is hydroxytoluene or butylated hydroxytoluene (BHT).

Preferably, the mixed but unset dental compositions of the invention will contain a combined weight of about 0.5 to about 40%, more preferably about 1 to about 30%, and most preferably about 5 to 20% water, solvents, diluents and comonomers, based on the total weight (including such water, solvents, diluents and comonomers) of the mixed but unset dental composition components.

The amount of inhibitor may be selected from 0.001 to 2% and preferably from 0.02 to 0.5% based on the total weight of the copolymer/comonomer/water mixture.

A polymerizable polymer according to the present invention, which is obtainable by the process as described above, is particularly useful for glass-ionomer cement (GIC) systems.

In case a dental composition according to the present invention is a glass ionomer cement, the dental composition may also include a modifying agent such as tartaric acid, for adjusting the working time and a setting time of the glass ionomer cement reaction, respectively, when preparing the cement as described in US-A 4,089, 830, US-A 4, 209,434, US-A 4,317, 681 and US-A 4,374, 936. In general, an increase in working time results in an increase in setting time as well.

The "working time" is the time between the beginning of the setting reaction when the polymer and modified particulate reactive filler are combined in the presence of water, and the time the setting reaction proceeds to the point when it is no longer practical to perform further physical work upon the system, e.g. spatulate it or reshape it, for its intended dental or medical application.

The "setting time" is the time measured from the beginning of the setting reaction in a restoration to the time sufficient hardening has occurred to allow subsequent clinical or surgical procedures to be performed on the surface of the restoration.

The composition of a preferred dental glass ionomer cement composition is as shown in the following table:

| Component in the dental cement | Percent by weight based on the total composition (preferred range) |
|---|---|
| Particulate reactive inorganic filler | 40-85 (50-70) |
| Polymerizable poly(ethylenimine) | 3-80 (5-20) |
| Water | 1-65 (5-45) |
| Polyacid | 0- 70 (0-50) |
| Additional filler | 0-20 (0-10) |
| Comonomer | 0-20 (5-10) |

The dental glass ionomer cement of the present invention is curable by a cement reaction, a radical polymerization and optionally by a further reaction. A further reaction may be a polyaddition reaction.

The dental glass ionomer composition may be a multi-pack, preferably a two-pack composition. The composition may be a paste/paste system, a powder/liquid system, or a liquid/paste system. The composition is designed so as to avoid premature curing of the components. For this purpose, the reactive inorganic filler component and any acid group containing component must be formulated so as to avoid a premature cement reaction. In a first embodiment, the reactive inorganic filler is contained in a first pack and any acid group containing component is contained in a second pack. The first pack may be a powder or a paste. The second pack may be a liquid or paste. In a second embodiment, the first pack is a powder comprising the reactive inorganic filler and a solid polyacid such as polyacrylic acid, and the second pack is a paste or liquid and contains a further acid group containing component.

The present invention will now be further illustrated based on the following examples.

### Examples

### Example 1 - Preparation of l-PEI

| | | |
|---|---|---|
| 20 g | 200 mmol | poly(2-ethyl-2-oxazolin) (PEO) |
| 32 mL | | concentrated hydrochloric acid |
| 80 mL | | distilled water |

20 g (200 mmol) PEO are dissolved in 80 mL water in a 250 mL two-neck flask equipped with a reflux condenser and a stopper. 32 mL concentrated hydrochloric acid are added and stirring is continued for 48 hours under reflux.

The hydrolyzed product is precipitated in acetone, neutralized in 5N sodium hydroxide and exhaustively dialyzed against water (MWCO= 1000 g/mol). Ultimately, the solution is lyophilized for a yellow highly viscous solid.
**¹H-NMR** (300 MHz, D₂O): *δ* (ppm)= 2.97 (4H, -CH₂-CH₂-), 2.11 (2H, -NH-CH₂-, salt), 0.97 (3H, -CH₃, salt).
**FT-IR:** *vₘₐₓ* [cm⁻¹]= 3372, 3284 (-NH-), 2963, 2839, 1606, 1552, 1459, 1401, 1367, 1291, 1136, 1072, 873, 750.

### Example 2 - Modification of b-PEI with succinic anhydride and methacrylic anhydride

| | | |
|---|---|---|
| 0.5 g | 11.4 mmol | *branched poly*(ethylenimine) (b-PEI) |
| 0.09 g | 0.6 mmol | methacrylic anhydride |
| 1.08 g | 10.8 mmol | succinic anhydride |
| 15 mL | | methanol |
| 1.15 g | 11.4 mmol | triethylamin |

500 mg (11.4 mmol) *b*-PEI are dissolved under ice-cooling in 15 mL methanol. Subsequently, 1.15 g (11.4 mmol) triethylamine is added. Then, 90 mg (0.6 mmol) methacrylic anhydride are added followed by 1.08 g (10.8 mmol) succinic anhydride. Stirring is continued overnight at room temperature.

Subsequently, methanol is evaporated, the residue is dissolved in water and acidified prior to exhaustive dialysis against water (MWCO=1000 g/mol). The solution obtained is lyophilized for obtaining a yellow solid.
**¹H-NMR** (300 MHz, D₂O): *δ* (ppm)= 5.65 (1H, >C=CH₂), 5.40 (1H, >C=CH₂), 4.00-2.50 (4H, backbone), 2.38 (4H, succinic anhydride), 1.85 (3H, -CH₃).
**FT-IR:** *vₘₐₓ* [cm⁻¹]= 3281, 3065, 2953, 2837, 1696 (-COOH), 1626 (-CONH- I), 1549 (-CONH- II), 1393, 1299, 1216, 1171, 948.

### Example 3 - Modification of l-PEI with succinic anhydride and methacrylic anhydride

| | | |
|---|---|---|
| 7.0 g | 162.79 mmol | *linear* po/y(ethylenimine) (*l*-PEI) |
| 15.48 g | 154.65 mmol | succinic anhydride |
| 1.25 g | 8.14 mmol | methacrylic anhydride |
| 16.47 g | 162.79 mmol | triethylamine |
| 150 mL | | methanol |

7.0 g (162.79 mmol) *l*-PEI is dissolved under ice-cooling in 150 mL methanol. Subsequently, 16.47 g (162.79 mmol) triethylamine is added. Then, 1.25 g (8.14 mmol) methacrylic anhydride are added followed by 15.48 g (154.65 mmol) succinic anhydride. Stirring is continued overnight at room temperature.

Subsequently, methanol is evaporated, the residue is dissolved in water and acidified prior to exhaustive dialysis against water (MWCO=1000 g/mol). The solution obtained is lyophilized for obtaining a yellow solid.
**¹H-NMR** (300 MHz, D₂O): *δ* (ppm)= 5.70 (1H, >C=CH₂), 5.43 (1H, >C=CH₂), 3.89-2.60 (4H, backbone), 2.55-2.44 (4H, succinic anhydride), 1.83 (3H, -CH₃).

## Claims

1. Dental composition comprising
(i) a polymerizable poly(ethylenimine) derivative, which contains repeating units of the following formula (I) 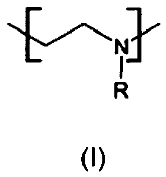 wherein the R, which are independent from each other, are selected from one or more groups of the following formula (II): 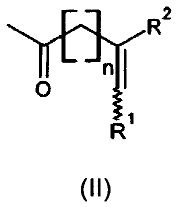 wherein
R¹ is a hydrogen atom, an alkyl group, a group -(CH₂)ₒCOOM', wherein M' is a hydrogen atom or a monovalent cation, and o is an integer of from 0 to 6;
R² is a hydrogen atom, an alkyl group, or a group -(CH₂)ₚCOOM", wherein M" is a hydrogen atom or a monovalent cation, and p is an integer of from 0 to 6;
n is 0 or 1;
and optionally a hydrogen atom, a further repeating unit of formula (I), and one or more groups of the following formula (III): 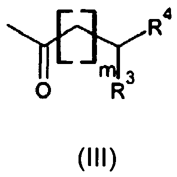
wherein
R³ is a group -(CH₂)_{q}COOM"', wherein M"' is a hydrogen atom or a monovalent cation, and q is an integer of from 0 to 6;
R⁴ is a hydrogen atom, an alkyl group, or a group -(CH₂)ᵣCOOM"", wherein M"" is a hydrogen atom or a monovalent cation, and r is an integer of from 0 to 6;
m is an integer of 0 to 4;
provided that when none of R in any of the repeating groups of formula (I) of the polymerizable poly(ethylenimine) derivative is a group of formula (II) wherein R¹ is a group -(CH₂)ₒCOOM' or R² is a group -(CH₂)ₚCOOM", then at least one R must be a group of formula (III);
(ii) an initiator system;
(iii) water; and
(iv) optionally a particulate filler.

2. The dental composition according to claim 1, wherein R¹ is a hydrogen atom.

3. The dental composition according to claim 1 or 2, wherein R² is an alkyl group.

4. The dental composition according to any one of the preceding claims, wherein n is 0.

5. The dental composition according to any one of the preceding claims, wherein n is 1.

6. The dental composition according to any one of claims 4 or 5, wherein R¹ is a group -(CH₂)ₚCOOM, wherein M is a hydrogen atom or a metal atom, and p is an integer of from 0 to 6.

7. The dental composition according to any one of claims 4 to 6, wherein R² is a group -(CH₂)ₚCOOM, wherein M is a hydrogen atom or a metal atom, and p is an integer of from 0 to 6.

8. The dental composition according to any one of the preceding claims, wherein the average amount of repeating units of the formula (I), wherein R is one or more groups of the formula (II), in the polymerizable poly(ethylenimine) derivative is from 0.1 to 70 mol% based on the total amount of repeating units of the formula (I).

9. The dental composition according to any one of the preceding claims, which is selected from restorative and filling materials, luting cements, adhesive cements, base or orthodontic cements, cavity liners and bases, and pit and fissure sealants.

10. A process for preparing a dental composition which comprises reacting a poly(ethylenimine) derivative, which contains repeating units of the following formula (I) 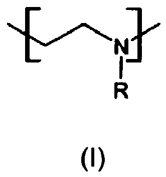 wherein R is a hydrogen atom with a compound of the following formula (IV) 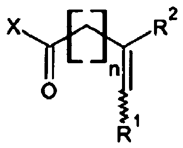 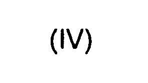 wherein R¹, R² and n are as defined in claim 1 and X is a leaving group or forms together with R¹ or R² a cyclic carboxylic anhydride, and optionally with a compound of the following formula (V) 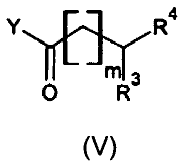 wherein R³, R⁴ and m are as defined in claim 1 and Y is a leaving group or forms together with R³ or R⁴ a cyclic carboxylic anhydride.

11. The process according to claim 10, wherein the poly(ethylenimine) derivative, which contains repeating units of the formula (I) is a linear polyethyleneimine obtainable by hydrolyzing poly(2-ethyl-2-oxazolin).

12. The process according to any one of claims 9 or 10, wherein the compound of formula (IV) is methacrylic anhydride.
